# EUROPEAN PATENT APPLICATION

(11) **EP 3 656 379 A1**
(43) Date of publication of application: **27.05.2020**
(21) Application number: 19217154.4
(22) Date of filing: 05.12.2014
(51) Int. Cl.: A61K 9/16, A61K 9/28, A61K 31/225

(54) **PHARMACEUTICAL COMPOSITIONS COMPRISING DIMETHYL FUMARATE**

(30) Priority: 12.12.2013 EP 13382504
(62) Divisional of application: 14809821.3
(71) Applicant: Almirall S.A., 08022 Barcelona (ES)
(72) Inventor: PLANELLS JIMENEZ, Maria, E-08980 Barcelona (ES); DUARTE LOPEZ, Begoña, E-08980 Barcelona (ES); GUIRO COLL, Pere, E-08980 Barcelona (ES)
(74) Representative: J A Kemp LLP

(57) **Abstract**

Pharmaceutical compositions are described comprising (a) dimethyl fumarate, (b) a diluent selected from monosaccharides, disaccharides, starch and starch derivatives, calcium and magnesium inorganic salts, sugar alcohols, and mixtures thereof, (c) microcrystalline cellulose and (d) croscarmellose sodium, wherein the dimethyl fumarate is not covered with a gastro-resistant coating. These compositions are intended for the treatment of some inflammatory autoimmune diseases or disorders.

## Description

### FIELD OF THE INVENTION

The present invention relates to pharmaceutical compositions comprising (a) dimethyl fumarate, (b) a diluent selected from monosaccharides, disaccharides, starch and starch derivatives, calcium and magnesium inorganic salts, sugar alcohols, and mixtures thereof, (c) microcrystalline cellulose and (d) croscarmellose sodium, wherein the dimethyl fumarate is not covered with a gastro-resistant coating.

The invention further relates to methods of treatment of some inflammatory autoimmune diseases or disorders by administering these compositions.

### BACKGROUND OF THE INVENTION

Inflammatory or autoimmune diseases or disorders such as rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis, are a major health burden especially in industrialized countries. These disorders in general cannot be cured, but the condition can be controlled or reduced in many cases.

Fumaric acid esters (FAE) are chemical compounds derived from unsaturated dicarboxylic fumaric acid and have been used in the treatment of psoriasis for years, originally proposed by the German chemist Walter Schweckendiek.

In 1994, Fumaderm® (Fumapharm AG), a mixture of dimethyl fumarate (DMF) and calcium, magnesium and zinc salts of monoethyl fumarate (MEF), was approved for the treatment of psoriasis in Germany. Fumaderm® is available in two different dosage strengths: low strength tablets (Fumaderm® initial) containing 30 mg dimethylfumarate, 67 mg Ca-ethylhydrogenfumarate, 5 mg Mg-ethylhydrogenfumarate and 3 mg Zn-ethylhydrogenfumarate; and high strength tablets (Fumaderm®) containing 120 mg dimethylfumarate, 87 mg Ca-ethylhydrogenfumarate, 5 mg Mg-ethylhydrogenfumarate and 3 mg Zn-ethylhydrogenfumarate.

Both Fumaderm® initial and Fumaderm® are enteric-coated tablet containing the following excipients: croscarmellose sodium, talc, magnesium stearate, coloring agent E171 and E132 (only in Fumaderm®), methacrylic acid-methylmethacrylate-copolymer (1:1), methacrylic acid-ethylacrylate- copolymer (1:1), Macrogol 6000, simethicone, povidone, triethyl citrate, microcrystalline cellulose, and highly disperse silicon dioxide.
In addition, the tablets should be stored not above 25°C. (Fumaderm® initial / Fumaderm®; Summary of Product Characteristics, version February 2009).

FAE therapy is associated with adverse events such as gastrointestinal complaints, flushing or decreasing in lymphocyte counts. In order to improve safety and efficacy of Fumaderm®, guidelines for the treatment of severe psoriasis with FAE were established in 1999 (Mrowietz U. et al, British Journal of Dermatology 1999, 141, 424-429). The two dosage strengths of Fumaderm® are intended to be applied in an individually base dosage regime starting with Fumaderm® initial in an escalating dose, and after some weeks of treatment, e.g. three weeks, switching to Fumaderm®.

In March 2013, Tecfidera® (Biogen Idec), dimethyl fumarate delayed-release capsules for oral use, was approved by the US FDA for the treatment of patients with relapsing forms of multiple sclerosis.
Tecfidera® is provided as hard gelatin delayed-release capsules for oral administration, containing 120 mg or 240 mg of dimethyl fumarate and containing the following excipients: microcrystalline cellulose, silicified microcrystalline cellulose, croscarmellose sodium, talc, silica colloidal silicon dioxide, magnesium stearate, triethyl citrate, methacrylic acid copolymer - Type A, methacrylic acid copolymer dispersion, simethicone (30% emulsion), sodium lauryl sulphate, and polysorbate 80. In addition, the capsules should be stored at 15 to 25°C (Tecfidera® approved labelling text dated March 27, 2013).
Tecfidera® has to be administered in an escalating dose, 120 mg for the 7 first days twice daily and, after that, the 240 mg dose twice daily.

The appearance of a solid dosage form, i.e. tablet, capsule, etc., is of great importance to the way that it is perceived by the patient. The macroscopic appearance of a tablet, its shape, size, colour and markings can all contribute to the patients' expectations of a medicine, while the microscopic appearance, such as surface roughness and colour homogeneity, are easily perceived as measures of quality. Typically, new products are identified because of their unique appearance achieved by a combination of shape, size, colour and surface markings.

Fumaderm® and Tecfidera® have a dosage regime starting with the lower dose and after some days/weeks of treatment, switching to the higher dose. In both cases, the compositions of the two strengths are qualitatively and quantitatively different and, therefore, having independent manufacturing process, which increases the manufacturing cost.

In addition, the only way for the patients to distinguish the low and high doses is through colour, which is a limitation for visually impaired people.

Furthermore, Fumaderm® and Tecfidera® present restrictive storage conditions. Storage specifications which are restrictive can add to the economic cost of the product, reduce its shelf life, inconvenience the pharmacist, discourage pharmacies from stocking a product and adversely affect product performance once it is in the patient's home.

Hence, there exists a need to overcome the aforementioned drawbacks of having pharmaceutical compositions comprising fumaric acid esters, in particular dimethyl fumarate, with restrictive storage conditions and with different strengths, which are qualitatively and quantitatively different and, which can only be distinguished by the patient in terms of colour.

Pharmaceutical compositions comprising dimethyl fumarate are known in the art. For instance, EP 0 312 697 describes pharmaceutical compositions in the form of enteric coated tablets comprising dimethyl fumarate and the calcium, magnesium and zinc salts of monoethylfumarate.

US 6,355,676 and US 6,509,376 describe pharmaceutical compositions in the form of enteric-coated micro-tablets or micro-pellets comprising dimethyl fumarate, where the size or the mean diameter, respectively, of the pellets or micro-tablets is in the range from 300 to 2,000 µm, especially in the range of 500 or 1,000 µm. According to US 6,355,676 and US 6,509,376 the micro-tablets may present an improved tolerance in the gastrointestinal tract versus conventional tablets.

WO 2010/126605 describes pharmaceutical compositions comprising dimethyl fumarate in the form of capsules containing enteric-coated micro-tablets.

WO 2006/037342, WO 2007/042034, WO 2010/079221 and WO 2010/079222 describe controlled release pharmaceutical compositions comprising as an active substance one or more fumaric acid esters selected from di-(C₁-C₅)alkylesters of fumaric acid, i.e. dimethyl fumarate, and mono-(C₁-C₅)alkylesters of fumaric acid. In particular, these documents describe that the fumaric acid esters, particularly dimethyl fumarate, are in the form of granules, particles or (micro-)crystals which are covered with a gastro-resistant coating previous to be manufactured to tablets or capsules, which are latter on enteric coated. This coating of the dimethyl fumarate granules, particles or (micro-)crystals allows that the active ingredient be delivered in a manner that is prolonged, slow and/or delayed compared to Fumaderm®.

WO 2013/076216 describes particles of dimethyl fumarate coated by at least one layer comprising a pharmaceutically acceptable pH-dependent enteric-resistant polymer.

WO 2013/119677 describes pharmaceutical compositions comprising dimethyl fumarate wherein the total amount of dimethyl fumarate in the composition ranges from 43 to 95 wt.%.

However, none of these documents describe a bulk pharmaceutical composition which could be used to obtain different pharmaceutical compositions with different dimethyl fumarate strengths, i.e. homothetic tablets, having the same qualitative composition and maintaining the same wt.% of all the components, only varying the tablet mass.

### SUMMARY OF THE INVENTION

The present application refers to pharmaceutical compositions comprising (a) dimethyl fumarate, (b) a diluent selected from monosaccharides, disaccharides, starch and starch derivatives, calcium and magnesium inorganic salts, sugar alcohols, and mixtures thereof, (c) microcrystalline cellulose and (d) croscarmellose sodium, wherein the dimethyl fumarate is not covered with a gastro-resistant coating.

The authors of the present invention have surprisingly found that, a pharmaceutical composition as defined above can be used as a bulk pharmaceutical composition which allows obtaining pharmaceutical compositions for oral administration with different dimethyl fumarate strength, i.e. homothetic tablet cores (previous to coating), having the same qualitative composition and maintaining the same wt.% of all the components, only varying the tablet core mass. This is of particular interest to differentiate the different strengths by the size of the tablet and not only by the colour, which is in favour of visually impaired people.

In addition, a bulk pharmaceutical composition is helpful in terms of reducing the manufacturing processes and, at the same time, manufacturing costs.

Furthermore, the pharmaceutical composition of the invention presents improved storage conditions, i.e., the storage conditions of the pharmaceutical composition of the invention are less restrictive than the conditions indicated in the prescribing information for Fumaderm® and Tecfidera®.

Finally, despite having reduced the number of active ingredients from Fumaderm® and having different excipients, the pharmaceutical composition of the invention presents a dissolution profile similar to Fumaderm®.

The invention further relates to a pharmaceutical composition as defined above for use in the treatment or prevention of inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis; in particular for use in the treatment or prevention of multiple sclerosis or psoriasis.

The invention further relates to the use of a pharmaceutical composition as defined above for the manufacture of a medicament for the treatment or prevention of inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis; in particular for the treatment or prevention of multiple sclerosis or psoriasis.

The invention further relates to a method for treating a subject afflicted with inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis; which comprises administering to said subject a therapeutically effective amount of a pharmaceutical composition as defined above; in particular for treating a subject afflicted with multiple sclerosis or psoriasis.

### DETAILED DESCRIPTION OF THE INVENTION

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The term "therapeutically effective amount" of a compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

The term "not covered with a gastro-resistant coating" as used herein means that dimethyl fumarate particles are not previously coated with pharmaceutical acceptable polymers such as ethylcellulose, methacrylic/acrylic acid copolymers or ammonio methacrylate copolymers (such as ammonio methacrylate copolymer type A or B , or methacrylic acid copolymer A or B), polyvinyl acetate polymer, methacryl-ethylacetate polymer; or hydrophilic excipients such as polyethylene glycol (PEG), povidone, hydroxyl propyl cellulose (HPC), hydroxyethyl starch (HES) or hydroxypropyl methyl cellulose (HPMC).

### (a) Dimethyl fumarate

Dimethyl fumarate (Dimethyl (E)-butenedioate; CAS RN 624-49-7) is the methyl ester of fumaric acid, which presents the molecular formula C₆H₈O₄ and the molecular mass 144.13 g/mol and the following chemical formula

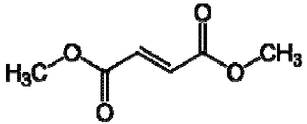

According to the German Medicines Codex 2004 (DAC 2004) it is a white crystalline powder having a melting point in the range from 102-105°C.
The crystallographic properties of dimethyl fumarate are described Kooijman H et al, Acta Cryst. (2004), E60, o917-o918.
Dimethyl fumarate can be obtained by reacting fumaric acid and methanol under the presence of concentrated sulphuric acid as catalyst (Ma Hongfei, Chemical industry Times, 2005, Vol. 19, No. 4, 18-19)
Typically, the dimethyl fumarate is sieved and/or milled to control its particle size. In a preferred embodiment the dimethyl fumarate has a particle size distribution d(10) between 5-20 µm, a d(50) between 30-70 µm, and a d(90) between 80-150 µm, measured using the laser diffraction particle size analyzer Mastersizer 2000 (Malvern Instruments).

### (b) The diluent

Diluents are fillers designated to make up the required bulk of the dosage form, i.e. tablet, when the drug dosage itself is inadequately to produce this bulk (The Theory and Practice of Industrial Pharmacy, 3rd edition, 1986, ISBN 0-8121-0977-5).
Typically, the diluent is selected from monosaccharides, disaccharides, starch and starch derivatives, calcium and magnesium inorganic salts, sugar alcohols, and mixtures thereof.

Monosaccharides are colourless, crystalline solids that are freely soluble in water but insoluble in nonpolar solvents. The simplest monosaccharides are the two three-carbon trioses: glyceraldehyde, an aldotriose, and dihydroxyacetone, a ketotriose. Monosaccharides with four, five, six, and seven carbon atoms in their backbones are called, respectively, tetroses, pentoses, hexoses, and heptoses (Lehninger Principles of Biochemistry, 4th edition). Suitable monosaccharides according to the invention are D-ribose, D-arabinose, D-xylose, D-glucose (dextrose), D-mannose, D-galactose and D-fructose.

Disaccharides consist of two monosaccharides joined covalently by an *O*-glycosidic bond, which is formed when a hydroxyl group of one sugar reacts with the anomeric carbon of the other (Lehninger Principles of Biochemistry, 4th edition). Suitable disaccharides according to the invention are lactose, maltose (α-D-glucopyranosyl-(1-4)-D-glucopyranose), sucrose (α-D-glucopyranosyl β-D-fructofuranoside) and trehalose (α-D-glucopyranosyl α-D-glucopyranoside).

Starch (CAS RN 9005-25-8; molecular formula (C₆H₁₀O₅)n where n = 300-1000) is a natural homopolysaccharide, which consists of linear amylose and branched amylopectin, two polysaccharides based on α-(D)-glucose. Both polymers are organized in a semicrystalline structure, and in the starch granule, amylopectin forms the crystalline portion. Starch occurs as an odourless and tasteless, fine, white to off-white powder. It consists of very small spherical or ovoid granules or grains whose size and shape are characteristic for each botanical variety (Handbook of Pharmaceutical Excipients, 6th edition, 2009; ISBN 9780853697923, UK).

Starch derivatives according to the invention refer to partially or fully pregelatinized starch (starch that has been chemically and/or mechanically processed to rupture all or part of the starch granules), hydrolized starch, dextrinized starch, alkaline-modified starch (with sodium hydroxide or potassium hydroxide), bleached starch (with hydrogen peroxide), enzyme-treated starch, monostarch phosphate, monostarch phosphate, hydroxypropylated starch, hydroxyethyl starch, octenyl succinic anhydride (OSA) starch, cationic starch (adding positive electrical charge to starch), carboxymethylated starch (with monochloroacetic acid adding negative charge), or mixtures thereof.

Calcium and magnesium inorganic salts according to the invention refer to calcium carbonate, dibasic calcium phosphate, calcium sulphate, magnesium carbonate, or mixtures thereof.

Sugar alcohols are polyalcohols resulting from the reduction of the carbonyl group in a monosaccharide to a hydroxyl group. They are white, water-soluble solids that occur naturally and are used widely in the food industry as thickeners and sweeteners. Suitable sugar alcohols according to the invention are erythritol, isomalt, lactitol, mannitol, maltitol, sorbitol and xylitol.

Preferably, the diluent (b) is selected from lactose, D-glucose (dextrose), sucrose (D-sucrose), fructose (D-fructose), galactose (D-galactose), starch, calcium carbonate, dibasic calcium phosphate, calcium sulphate, magnesium carbonate, isomalt, mannitol, maltitol, sorbitol, xylitol, and mixtures thereof.

In a preferred embodiment, the diluent (b) is selected from fructose, lactose, dibasic calcium phosphate, mannitol, and mixtures thereof. In a more preferred embodiment, the diluent (b) is lactose.

### Fructose

Fructose (D-fructose) is a monosaccharide that occurs as odourless, colourless crystals or a white crystalline powder with a very sweet taste (CAS RN 57-48-7; molecular formula C₆H₁₂O₆; molecular weight 180.16 g/m). Fructose occurs naturally in honey and a large number of fruits. It may be prepared from inulin, dextrose, or sucrose by a number of methods. Commercially, fructose is mainly manufactured by crystallization from high-fructose syrup derived from hydrolyzed and isomerized cereal starch or cane and beet sugar (Handbook of Pharmaceutical Excipients, 6th edition, 2009).

### Lactose

Lactose is a natural monosaccharide, obtained from milk, which consist of one galactose and one glucose moiety. Lactose occurs as white to off-white crystalline particles or powder. It is odourless and slightly sweet-tasty. The Handbook of Pharmaceutical Excipients, 6th edition, 2009, describes different lactose types which are suitable according to the current invention: anhydrous lactose (CAS RN 63-42-3; molecular formula C₁₂H₂₂O₁₁; molecular weight 342.30 g/m), inhalation lactose, lactose monohydrate (CAS RN 5989-81-1; molecular formula C₁₂H₂₂O₁₁.H₂O; molecular weight 360.31 g/m), and spray-dried lactose, which is a mixture of amorphous lactose (1:1 mixture of α-and-β-lactose) and lactose monohydrate.

In a preferred embodiment, the lactose is selected from lactose monohydrate or spray-dried lactose, preferably spray-dried lactose.

Typically, the spray-dried lactose has a bulk density between 0.55 and 0.68 g/cm³ and a tapped density between 0.65 and 0.75 g/cm³. In a preferred embodiment the spray-dried lactose has a particle size distribution (retained on air jet sieve, cumulative) 75 µm (US standard #200) 60-80%, 106 µm (US standard #140) 30-55% and 250 µm (US standard #60) 0.0-0.5%.

### Dibasic calcium phosphate

Dibasic calcium phosphate according to the invention refers to anhydrous dibasic calcium phosphate and dibasic calcium phosphate hydrate.

Anhydrous dibasic calcium phosphate (calcium phosphate, dibasic anhydrous; CAS RN 7757-93-9; molecular formula CaHPO₄; molecular weight 136.06 g/m) is a white, odourless, tasteless powder or crystalline solid, which occurs as triclinic crystals (Handbook of Pharmaceutical Excipients, 6th edition, 2009).

Dibasic calcium phosphate hydrate (calcium phosphate, dibasic dihydrate; CAS RN 7789-77-7; molecular formula CaHPO₄.2H₂O; molecular weight 172.09 g/m) is a white, odourless, tasteless powder or crystalline solid, which occurs as monoclinic crystals (Handbook of Pharmaceutical Excipients, 6th edition, 2009).

In a preferred embodiment, the dibasic calcium phosphate is anhydrous dibasic calcium phosphate.

### Mannitol

Mannitol (D-mannitol; CAS RN 69-65-8; molecular formula C₆H₁₄O₆; molecular weight 182.17 g/m) is a hexahydric alcohol related to mannose and is isomeric with sorbitol. Mannitol occurs as a white, odourless, crystalline powder, or free flowing granules. It has a sweet taste, approximately as sweet as glucose and half as sweet as sucrose, and imparts a cooling sensation in the mouth. The Handbook of Pharmaceutical Excipients, 6th edition, 2009, describes different commercially available grades of mannitol which are suitable according to the current invention such as Pearlitol 300 DC, Pearlitol 400 DC and Pearlitol 500 DC; all manufactured by Roquette Freres.

### (c) Microcrystalline cellulose

Microcrystalline cellulose (CAS RN 9004-34-6; molecular formula (C₆H₁₀O₅)ₙ where n is approx. 220; molecular weight approx. 36000 g/m) is a purified, partially depolymerized cellulose that occurs as a white, odourless, tasteless, crystalline powder composed of porous particles (Handbook of Pharmaceutical Excipients, 6th edition, 2009).

Typically, the microcrystalline cellulose has a bulk density between 0.28 and 0.33 g/cm³. In a preferred embodiment the microcrystalline cellulose has a particle size distribution d(10) between 25-50 µm, a d(50) between 100-150 µm, and a d(90) between 195-280 µm, measured using a laser diffraction particle size analyzer Mastersizer (Malvern Instruments).

### (d) Croscarmellose sodium

Croscarmellose sodium (cellulose, carboxymethyl ether, sodium salt, crosslinked; CAS RN 74811-65-7) is a crosslinked polymer of carboxymethyl cellulose sodium. Croscarmellose sodium occurs as an odourless, white or grayish white powder (Handbook of Pharmaceutical Excipients, 6th edition, 2009).

Typically, the croscarmellose sodium has a bulk density around 0.529 g/cm³ and a tapped density between around 0.819 g/cm³. In a preferred embodiment the microcrystalline cellulose has a particle size distribution d(10) of not more than 25 µm, a d(50) between 25-55 µm, and a d(90) of not less than 60 µm, measured using a laser diffraction particle size analyzer Mastersizer (Malvern Instruments).

### The pharmaceutical compositions

Typically, the present invention provides pharmaceutical compositions comprising (a) dimethyl fumarate, (b) a diluent selected from monosaccharides, disaccharides, starch and starch derivatives, calcium and magnesium inorganic salts, sugar alcohols, and mixtures thereof, (c) microcrystalline cellulose and (d) croscarmellose sodium, wherein the dimethyl fumarate is not covered with a gastro-resistant coating.

Preferrably, the diluent (b) is selected from lactose, D-glucose (dextrose), sucrose, fructose, galactose, starch, calcium carbonate, dibasic calcium phosphate, calcium sulphate, magnesium carbonate, isomalt, mannitol, maltitol, sorbitol, xylitol, and mixtures thereof.

In a preferred embodiment, the diluent (b) is selected from lactose, dibasic calcium phosphate, isomalt, mannitol, and mixtures thereof. In a more preferred embodiment, the diluent (b) is lactose.

In a particular preferred embodiment, the present invention provides pharmaceutical compositions comprising (a) dimethyl fumarate, (b) lactose, (c) microcrystalline cellulose and (d) croscarmellose sodium.

In a preferred embodiment, the pharmaceutical composition as defined above further comprises (e) at least one glidant.

Typically, the glidant (e) is selected from calcium phosphate, calcium silicate, powdered cellulose, magnesium silicate, magnesium trisilicate, magnesium carbonate, magnesium oxide, magnesium lauryl sulphate, sodium lauryl sulphate starch, silicon dioxide, talc, colloidal silica, colloidal anhydrous silica (colloidal silicon dioxide or fumed silicon dioxide) and mixtures thereof.

Preferably, the glidant (e) is selected from colloidal silica anhydrous, talc, or a combination thereof, more preferably colloidal anhydrous silica.

In a preferred embodiment, the pharmaceutical composition as defined above further comprises (f) at least one lubricant.

Typically, the lubricant (f) is selected from magnesium stearate, calcium stearate, sodium stearyl fumarate, polyethylene glycol (in particular polyethylene glycol 4000 and 6000), sodium lauryl sulfate, magnesium lauryl sulfate, sodium benzoate, potassium benzoate, light mineral oil, hydrogenated vegetable oils (in particular hydrogenated castor oil), glycerin monostearate, glyceryl behenate, glyceryl palmitostearate, stearic acid, zinc stearate, and mixtures thereof.

Preferably, the lubricant (f) is magnesium stearate.

In a preferred embodiment, in the pharmaceutical composition as defined above the weight ratio (c) microcrystalline cellulose to (b) diluent is in the range from 2:5 to 5:2, preferably in the range from 2:1 to 1:2, more preferably 4:3.

In another preferred embodiment, in the pharmaceutical composition as defined above the weight ratio (c) microcrystalline cellulose to (c) dimethyl fumarate is in the range from 5:1 to 1:5, preferably in the range from 3:1 to 1:3, more preferably 8:5.

In another preferred embodiment, in the pharmaceutical composition as defined above the weight ratio (b) diluent to (c) dimethyl fumarate is in the range from 5:1 to 1:5, preferably in the range from 3:1 to 1:3, more preferably 6:5.

In another preferred embodiment, in the pharmaceutical composition as defined above the weight ratio (c) microcrystalline cellulose to (d) croscarmellose sodium is in the range from 30:1 to 1:5, preferably in the range from 20:1 to 1:2, more preferably 10:1.

In another preferred embodiment, in the pharmaceutical composition as defined above the weight ratio (b) diluent to (d) croscarmellose sodium is in the range from 20:1 to 1:5, preferably in the range from 10:1 to 1:3, more preferably 15:2.

The pharmaceutical composition of the invention may optionally contain other conventional ingredients such as anti-oxidants, colorants, flavouring agents, preservatives and taste-masking agents.

In a preferred embodiment, the pharmaceutical composition of the invention is administered orally (peroral administration; per os (latin)).

Typically, the pharmaceutical composition of the invention is a solid dosage form, i.e. immediate-release tablet, immediate-release capsule, delayed-release tablet, delayed-release capsule, sustained-release tablet, sustained-release capsule, soluble tablet, dispersible tablet, effervescent tablet, chewable tablet, chewable gum, buccal tablet, sublingual tablet, orally disintegrating tablet, lozenge, pastille, hard gelatin capsule or soft gelatin capsule.

Preferably, the pharmaceutical composition of the invention is in the form of a delayed-release tablets, more preferably a gastro-resistant (enteric coated) tablet. Gastro-resistant tablets are delayed-release tablets that are intended to resist the gastric fluid and to release their active substance(s) in the intestinal media. Usually they are prepared from granules or particles already covered with a gastro-resistant coating or in certain cases by covering tablet cores with a gastro-resistant coating (enteric coated tablets) (European Pharmacopoeia 6.0, 2007, ISBN 9789287160546). In a preferred embodiment, the pharmaceutical composition of the invention is in the form of a gastro-resistant (enteric coated) tablet prepared by covering tablet cores with a gastro-resistant coating.

As previously indicated, the dimethyl fumarate is not covered with a gastro-resistant coating, i.e. dimethyl fumarate particles are not previously coated with pharmaceutical acceptable polymers or hydrophilic excipients when the tablet core (tablet previous to coating) is prepared.

Tablet blends may be compressed directly or by roller to form tablets. Tablet blends or portions of blends may alternatively be wet-, dry-, or melt-granulated, melt congealed, or extruded before tableting. The formulation of tablets is discussed in detail in Remington: The Science and Practice of Pharmacy, 21st Edition, 2005, ISBN 0781746736.

Coatings on tablet cores usually consist of a mixture of substances, for example, one or more plasticizers, one or core polymers, one or more copolymers, one or more glidants, one or more pigments, or mixtures thereof. Coating of tablet cores is discussed in detail in Pharmaceutical Manufacturing Handbook: Production and Processes, 2008, ISBN 9780470259580.

Examples of suitable plasticizers in the coating (compounds which reduce the minimum film-forming temperatures as well as the glass transition temperature) include one or more of acetyltributyl citrate, acetyltriethyl citrate, benzyl benzoate, cellulose acetate phthalate, chlorbutanol, dextrin, dibutyl phthalate, dibutyl secacate, diethyl phthalate, dimethyl phthalate, glycerin, glycerin monostearate, hypromellose phthalate, mannitol, mineral oil, lanolin alcohol, palmitic acid, polyethylene glycol, polyvinyl acetate phthalate, propylene glycol, 2-pyrrolidone, sorbitol, stearic acid, triacetin, tributyl citrate, triethanolamine, triethyl citrate, dibutyl sebacate, polyethylene glycol and propylene glycol.

Examples of suitable polymers in the coating include one or more of methacrylic acid polymers, acrylic polymers, hydroxypropyl methylcellulose (HPMC), hydroxypropyl cellulose acetate succinate (HPMCAS), ethylcellulose (EC), carboxymethyl ethylcellulose (CMEC), poly(vinyl alcohol) (PVA), and polyvinyl acetate phthalate (PVAP). These polymers are available as aqueous dispersions, powders or organic solutions (e.g. alcohols, acetone). An example of commercially available organic solution is a mixture of poly(vinyl alcohol), methacrylic acid copolymer-type C, polyethylene glycol, talc, a neutralizing agent and pigment; marketed under the trade name OPADRY ® 200 by Colorcon, Inc.

Examples of suitable copolymers in the coating include one or more of methacrylic acid-methyl methacrylate (50:50) copolymer, methacrylic acid-methyl methacrylate (30:70) copolymer, methacrylic acid-ethylacrylate (50:50) copolymer, or a methacrylic acid-methyl acrylate-methyl methacrylate copolymer. These copolymers are available as aqueous dispersions, powders or organic solutions (e.g. alcohols, acetone). Examples of commercially available copolymers include the methacrylic acid copolymers marketed under the trade name EUDRAGIT ® by Evonik, which include EUDRAGIT ® L 30 D-55 (methacrylic acid - ethyl acrylate copolymer (1:1), 30% aqueous dispersion), EUDRAGIT ® L 100-55 (methacrylic acid - ethyl acrylate copolymer (1:1), powder form), EUDRAGIT ® L 100 (methacrylic acid - methyl methacrylate copolymer (1:1), powder form), EUDRAGIT ® L 12,5 (methacrylic acid - methyl methacrylate copolymer (1:1), 12.5% organic solution), EUDRAGIT ® S 100 (methacrylic acid - methyl methacrylate copolymer (1:2), powder form), EUDRAGIT ® S 12,5 (methacrylic acid - methyl methacrylate copolymer (1:1), 12.5% organic solution) and EUDRAGIT ® FS 30 D (methacrylic acid - methyl acrylate - methyl methacrylate copolymer, 30% aqueous dispersion).

Examples of suitable glidants in the coating include talc or glycerol monostearate.

Examples of suitable pigments in the coating include titanium dioxide, aluminium lakes, indigo carmine lakes or iron oxide pigments.

In a particular embodiment, the coating is carried out by means of a coating formulation which comprises i) one or more polymers and/or copolymers, ii) one or more glidants, iii) one or more plasticizers, and iv) one or more pigments. Optionally, the coating formulation can comprise one or more antifoam agents.

In a particular embodiment, the pharmaceutical composition of the invention is in the form of a gastro-resistant (enteric coated) tablet and comprises, based on the total weight of the tablet previous to coating (tablet core):
(a) 20-30 wt.% of dimethyl fumarate (not previously covered with a gastro-resistant coating);
(b) 25-35 wt.% of a diluent selected from lactose, D-glucose (dextrose), sucrose, fructose, galactose, starch, calcium carbonate, dibasic calcium phosphate, calcium sulphate, magnesium carbonate, isomalt, mannitol, maltitol, sorbitol, xylitol, and mixtures thereof;
(c) 35-45 wt.% of microcrystalline cellulose;
(d) 1-10 wt.% of croscarmellose sodium.

In another particular embodiment, the pharmaceutical composition of the invention is in the form of a gastro-resistant (enteric coated) tablet and comprises, based on the total weight of the tablet previous to coating (tablet core):
(a) 20-30 wt.% of dimethyl fumarate (not previously covered with a gastro-resistant coating);
(b) 25-35 wt.% of a diluent selected from lactose, dibasic calcium phosphate, isomalt, mannitol, and mixtures thereof;
(c) 35-45 wt.% of microcrystalline cellulose;
(d) 1-10 wt.% of croscarmellose sodium.

In a particular preferred embodiment, the pharmaceutical composition of the invention is in the form of a gastro-resistant (enteric coated) tablet and comprises, based on the total weight of the tablet previous to coating (tablet core):
(a) 20-30 wt.% of dimethyl fumarate (not previously covered with a gastro-resistant coating);
(b) 25-35 wt.% of lactose;
(c) 35-45 wt.% of microcrystalline cellulose;
(d) 1-10 wt.% of croscarmellose sodium.

In a preferred embodiment, the pharmaceutical compositions in the form of a gastro-resistant (enteric coated) tablet as defined above further comprises (e) at least one glidant.

Preferably, the glidant (e) is selected from colloidal silica anhydrous, talc, or a combination thereof, more preferably colloidal anhydrous silica.

When the gastro-resistant (enteric coated) tablet as defined above comprises (e) at least one glidant, the glidant (or combination of glidants) is present in an amount ranging from 0.1 to 5 wt.%, preferably from 0.2 to 3 wt.%, more preferably from 0.1 to 1 wt.%, based on the total weight of the tablet previous to coating (tablet core).

In another preferred embodiment, the pharmaceutical composition in the form of a gastro-resistant (enteric coated) tablet as defined above further comprises (f) at least one lubricant.

Preferably, the lubricant (f) is magnesium stearate.

When the gastro-resistant (enteric coated) tablet as defined above comprises (f) at least one lubricant, the lubricant (or combination of lubricants) is present in an amount ranging from 0.1 to 10 wt.%, preferably from 0.2 to 5 wt.%, more preferably from 0.1 to 2 wt.%, based on the total weight of the tablet previous to coating (tablet core).

In a particular embodiment, the pharmaceutical composition of the invention is in the form of a gastro-resistant (enteric coated) tablet and comprises, based on the total weight of the tablet previous to coating (tablet core):
(a) 20-30 wt.% of dimethyl fumarate (not previously covered with a gastro-resistant coating);
(b) 25-35 wt.% of lactose;
(c) 35-45 wt.% of microcrystalline cellulose;
(d) 1-10 wt.% of croscarmellose sodium;
(e) 0.1-5 wt.% of at least one glidant; preferably selected from colloidal anhydrous silica, talc, or a combination thereof; more preferably colloidal silica anhydrous;
(f) 0.1-10 wt.% of at least one lubricant, preferably magnesium stearate.

In a preferred embodiment, the pharmaceutical composition of the invention is in the form of a gastro-resistant (enteric coated) tablet and comprises, based on the total weight of the tablet previous to coating (tablet core):
(a) 25 wt.% of dimethyl fumarate (not previously covered with a gastro-resistant coating);
(b) 30 wt.% of lactose;
(c) 40 wt.% of microcrystalline cellulose;
(d) 4 wt.% of croscarmellose sodium;
(e) 0.5 wt.% of at least one glidant; preferably selected from colloidal anhydrous silica, talc, or a combination thereof; more preferably colloidal silica anhydrous;
(f) 0.5 wt.% of at least one lubricant, preferably magnesium stearate.

In a particular preferred embodiment, the pharmaceutical composition of the invention is in the form of a gastro-resistant (enteric coated) tablet and comprises, based on the total weight of the tablet previous to coating (tablet core):
(a) 30 mg of dimethyl fumarate (not previously covered with a gastro-resistant coating);
(b) 36 mg of lactose;
(c) 48 mg of microcrystalline cellulose;
(d) 4.8 mg of croscarmellose sodium;
(e) 0.6 mg of colloidal anhydrous silica;
(f) 0.6 mg of magnesium stearate.

In another particular preferred embodiment, the pharmaceutical composition of the invention is in the form of a gastro-resistant (enteric coated) tablet and comprises, based on the total weight of the tablet previous to coating (tablet core):
(a) 120 mg of dimethyl fumarate (not previously covered with a gastro-resistant coating);
(b) 144 mg of lactose;
(c) 192 mg of microcrystalline cellulose;
(d) 19.2 mg of croscarmellose sodium;
(e) 2.4 mg of colloidal anhydrous silica;
(f) 2.4 mg of magnesium stearate.

In a preferred embodiment, when the pharmaceutical composition of the invention is in the form of a gastro-resistant (enteric coated) tablet, the tablet core does not contain further excipients than the described above, i.e. (b) diluent, (c) microcrystalline cellulose, (d) croscarmellose sodium and optionally (e) colloidal anhydrous silica; and (f) magnesium stearate.

The invention also relates to a pharmaceutical composition as defined above for use in the treatment or prevention of inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis; in particular for use in the treatment or prevention of multiple sclerosis or psoriasis.

The invention also provides the use of a pharmaceutical composition as defined above for the manufacture of a medicament for the treatment or prevention of inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis; in particular for the treatment or prevention of multiple sclerosis or psoriasis.

The invention further relates to a method for treating a subject afflicted with inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis; in particular for treating a subject afflicted with multiple sclerosis or psoriasis; which comprises administering to said subject a therapeutically effective amount of a pharmaceutical composition as defined in above.

The invention also relates to a process for manufacturing a pharmaceutical composition as described above, the process comprising the steps of:
i) mixing (a) dimethyl fumarate, wherein the dimethyl fumarate is not previously covered with a gastro-resistant coating, (b) a diluent selected from monosaccharides, disaccharides, starch and starch derivatives, calcium and magnesium inorganic salts, sugar alcohols, and mixtures thereof, (c) microcrystalline cellulose, (d) croscarmellose sodium, and optionally other pharmaceutical excipients, to form a homogenous blend; and
ii) optionally sieving the blend.
The invention also provides pharmaceutical compositions obtainable by this process.

In a preferred embodiment, the process for manufacturing a pharmaceutical composition as described above comprising the steps of:
i) mixing (a) dimethyl fumarate, wherein the dimethyl fumarate is not previously covered with a gastro-resistant coating, (b) a diluent selected from monosaccharides, disaccharides, starch and starch derivatives, calcium and magnesium inorganic salts, sugar alcohols, and mixtures thereof, (c) microcrystalline cellulose, (d) croscarmellose sodium, and (e) at least one glidant to form a homogenous blend;
ii) optionally sieving the blend;
iii) adding (e) at least one glidant to the previous blend and mixing the resulting blend; and
iv) optionally sieving the final blend.
The invention also relates to pharmaceutical compositions obtainable by this process.

The invention also relates to a process for manufacturing gastro-resistant (enteric-coated) tablets as described above, the process comprising the steps of:
i) tableting the blend described above to obtain tablet cores; and
ii) coating the tablet cores.
The invention also provides gastro-resistant (enteric-coated) tablets obtainable by this process.

The invention also provides a combination which comprises (1) a pharmaceutical composition as defined above and (2) a therapeutically effective amount of a compound that reduces or eliminates flushing.

Flushing is known as a side effect of fumaric acid esters since early clinical studies and has led to the discontinuation of treatment in some patients (Nieboer C et al., Journal of the American Academy of Dermatology, 1989, 20:4, 601-608); Nieboer C et al., Dermatologica, 1990, 181:33-37). Thus, "a compound that reduces or eliminates flushing" refers to the ability of a compound to reduce the severity of flushing when it occurs, or result in fewer flushing events than would otherwise occur. As used herein the term "therapeutically effective amount" means an amount sufficient to reduce the severity of flushing when it occurs, or result in fewer flushing events than would otherwise occur. An amount adequate to accomplish this is defined as a "therapeutically effective amount".

The active compounds in the combination may be administered together in the same pharmaceutical composition or in different compositions intended for separate, simultaneous, concomitant or sequential administration by the same or a different route.

Also provided is a product comprising (1) a pharmaceutical composition as defined above and (2) a therapeutically effective amount of a compound that reduces or eliminates flushing, as a combined preparation for simultaneous, separate or sequential use in the treatment of a human or animal patient suffering from or susceptible to a pathological condition or disease as defined above.

Also provided is a kit of parts comprising (1) a pharmaceutical composition as defined above, together with instructions for simultaneous, separate or sequential use in combination with (2) a therapeutically effective amount of a compound that reduces or eliminates flushing, for the treatment of a human or animal patient suffering from or susceptible to a pathological condition or disease as defined above.

Also provided is a package comprising (1) a pharmaceutical composition as defined above and (2) a therapeutically effective amount of a compound that reduces or eliminates flushing, for simultaneous, separate or sequential use in the treatment of a pathological condition or disease as defined above.

Typically, the compound that reduces or eliminates flushing (2) is selected from acetylsalicylic acid, laropripant ((-)-[(3R)-4-(4-chlorobenzyl)-7-fluoro-5-(methylsulfonyl)-1,2,3,4-tetrahydrocyclopenta[b]indol-3-yl]acetic acid), COX inhibitors, or a combination thereof.

As used herein, the term COX inhibitor refers to a compound that inhibits both the cyclooxygenase-1 enzyme and the cyclooxygenase-2 enzyme. In one embodiment, the compound has a cyclooxygenase-1 IC₅₀ value of less than about 200 µM, preferably of less than about 100 µM, more preferably of less than about 50 µM, even more preferably of less than about 20 µM, and a cyclooxygenase-2 IC₅₀ value of less than about 50 µM, preferably of less than 25 µM, more preferably of less than about 15 µM, even more preferably of less than about 2µM, in the human whole blood assay (as described in Brideau et al., Inflamm Res., 45: 68-74 (1996)) and also has a selectivity ratio of cyclooxygenase-2 inhibition over cyclooxygenase-1 inhibition (determined as the ratio IC₅₀ COX-1 / IC₅₀ COX-2) of at least 0.1, preferably of at least 0.5, more preferably of at least 1, even more preferably of at least 2, and most preferably of at least 10.

In an embodiment, the COX inhibitor is selected from the group consisting of oxicams, piroxicam, meloxicam, isoxicam, tenoxicam, sudoxicam, CP-14,304, salicylates, disalcid, benorylate, trilisate, safapryn, solprin, diflunisal, fendosal, acetic acid derivatives, aceclofenac, diclofenac, fenclofenac, indomethacin, sulindac, tolmetin, isoxepac, furofenac, tiopinac, zidometacin, acematacin, fentiazac, zomepirac, clindanac, oxepinac, felbinac, etodolac, ketorolac, fenamates, mefenamic, meclofenamic, flufenamic, niflumic, tolfenamic acids, propionic acid derivatives, acetaminophen (paracetamol), ibuprofen, naproxen, benoxaprofen, flurbiprofen, ketoprofen, piketoprofen, fenoprofen, fenbufen, indopropfen, pirprofen, carprofen, oxaprozin, pranoprofen, miroprofen, tioxaprofen, suprofen, alminoprofen, tiaprofen, pyrazoles, phenylbutazone, oxyphenbutazone, feprazone, azapropazone, trimethazone, apricoxib, celecoxib, cimicoxib, deracoxib, etoricoxib, lumiracoxib, parecoxib sodium, rofecoxib, selenocoxib-1, valdecoxib, 2-(3,4-difluoro-phenyl)-4-(3-hydroxy-3-methyl-butoxy)-5-(4-methanesulfonyl-phenyl)-2H-pyridazin-3-one (ABT-963), 4-(4-Cyclohexyl-2-methyloxazol-5-yl)-2-fluorobenzenesulfonamide (JTE-522), N-[2-Cyclohexyloxy-4-nitrophenyl]methanesulfonamide (NS 398), (E)-(5)-(3,5-Di-tert-butyl-4-hydroxybenzylidene)-2-ethyl-1,2-isothiazolidine-1,1-dioxide (S-2474), 5(R)-thiosulfonamide-3(2H)-benzofuranone (SVT-2016), N-[7-[(methanesulfonyl)amino]-4-oxo-6-phenoxy-4H-1-benzopyran-3-yl] formamide (T-614), BMS-347070 (Bristol Myers Squibb), GSK-644784 (GlaxoSmithKline), RS 57067 (Roche Bioscience), SC-75416 (Pfizer), SC-58125 (Pfizer), SD-8381, 4-Methyl-2-(3,4-dimethylphenyl)-1-(4-sulfamoyl-phenyl)-1H-pyrrole, 2-(4-Ethoxyphenyl)-4-methyl-1-(4-sulfamoylphenyl)-1H-pyrrole, 3-(2,4-difluorophenoxy)-6-methyl-2-(4-(methylsulfonyl)phenyl)-4H-pyran-4-one, 3-(4-chlorophenoxy)-6-methyl-2-(4-(methylsulfonyl)phenyl)-4H-pyran-4-one, 3-(4-bromo-2-fluorophenoxy)-6-methyl-2-(4-(methylsulfonyl)phenyl)-4H-pyran-4-one, 3-(4-chloro-2-fluorophenoxy)-6-methyl-2-(4-(methylsulfonyl)phenyl)-4H-pyran-4-one, 3-(2,4-difluorophenoxy)-6-methyl-2-(4-(methylsulfinyl)phenyl)-4H-pyran-4-one, 3-(4-chloro-2-fluorophenoxy)-6-methyl-2-(4-(methylsulfinyl)phenyl)-4H-pyran-4-one, (S)-3-(2,4-difluorophenoxy)-6-methyl-2-(4-(methylsulfinyl)phenyl)-4H-pyran-4-one, (S)-3-(4-bromo-2-fluorophenoxy)-6-methyl-2-(4-(methylsulfinyl)phenyl)-4H-pyran-4-one, (S)-3-(4-chloro-2-fluorophenoxy)-6-methyl-2-(4-(methylsulfinyl)phenyl)-4H-pyran-4-one, (R)-3-(2,4-difluorophenoxy)-6-methyl-2-(4-(methylsulfinyl)phenyl)-4H-pyran-4-one, (R)-3-(4-bromo-2-fluorophenoxy)-6-methyl-2-(4-(methylsulfinyl)phenyl)-4H-pyran-4-one, (R)-3-(4-chloro-2-fluorophenoxy)-6-methyl-2-(4-(methylsulfinyl)phenyl)-4H-pyran-4-one, 4-(3-(2-fluorophenyl)-2-oxo-2,3-dihydrooxazol-4-yl)benzenesulfonamide, 4-(2-oxo-3-m-tolyl-2,3-dihydrooxazol-4-yl)benzenesulfonamide, 4-(2-oxo-3-p-tolyl-2,3-dihydrooxazol-4-yl)benzenesulfonamide, (R)-4-(4-(methylsulfinyl)phenyl)-3-phenylfuran-2(5H)-one, (S)-4-(4-(methylsulfinyl)phenyl)-3-phenylfuran-2(5H)-one, 4-(4-(methylsulfinyl)phenyl)-3-phenylfuran-2(5H)-one, 5-chloro-6'-methyl-3-(4-(methylsulfinyl)phenyl)-2,3'-bipyridine, (S)-5-chloro-6'-methyl-3-(4-(methylsulfinyl)phenyl)-2,3'-bipyridine, (R)-5-chloro-6'-methyl-3-(4-(methylsulfinyl)phenyl)-2,3'-bipyridine, and their pharmaceutically acceptable salts, their solvates, their N-oxides, their stereoisomers or their deuterated derivates thereof.

In a preferred embodiment, the COX inhibitor is selected from the group consisting of aceclofenac, diclofenac, acetaminophen (paracetamol), ibuprofen, naproxen, apricoxib, celecoxib, cimicoxib, deracoxib, etoricoxib, lumiracoxib, parecoxib sodium, rofecoxib, selenocoxib-1, valdecoxib, 2-(3,4-difluoro-phenyl)-4-(3-hydroxy-3-methyl-butoxy)-5-(4-methanesulfonyl-phenyl)-2H-pyridazin-3-one (ABT-963), 4-(4-Cyclohexyl-2-methyloxazol-5-yl)-2-fluorobenzenesulfonamide (JTE-522), N-[2-Cyclohexyloxy-4-nitrophenyl]methanesulfonamide (NS 398), (E)-(5)-(3,5-Di-tert-butyl-4-hydroxybenzylidene)-2-ethyl-1,2-isothiazolidine-1,1-dioxide (S-2474), 5(R)-thiosulfonamide-3(2H)-benzofuranone (SVT-2016), N-[7-[(methanesulfonyl)amino]-4-oxo-6-phenoxy-4H-1-benzopyran-3-yl] formamide (T-614), 3-(2,4-difluorophenoxy)-6-methyl-2-(4-(methylsulfonyl)phenyl)-4H-pyran-4-one, 4-(3-(2-fluorophenyl)-2-oxo-2,3-dihydrooxazol-4-yl)benzenesulfonamide, 4-(2-oxo-3-m-tolyl-2,3-dihydrooxazol-4-yl)benzenesulfonamide, 4-(2-oxo-3-p-tolyl-2,3-dihydrooxazol-4-yl)benzenesulfonamide, and their pharmaceutically acceptable salts, their solvates, their N-oxides, their stereoisomers or their deuterated derivates thereof.

In a further preferred embodiment, the COX inhibitor is selected from the group consisting of aceclofenac, diclofenac, acetaminophen (paracetamol), ibuprofen, naproxen, apricoxib, celecoxib, cimicoxib, deracoxib, etoricoxib, lumiracoxib, parecoxib sodium, rofecoxib, selenocoxib-1, valdecoxib, 3-(2,4-difluorophenoxy)-6-methyl-2-(4-(methylsulfonyl)phenyl)-4H-pyran-4-one, 4-(3-(2-fluorophenyl)-2-oxo-2,3-dihydrooxazol-4-yl)benzenesulfonamide, 4-(2-oxo-3-m-tolyl-2,3-dihydrooxazol-4-yl)benzenesulfonamide, 4-(2-oxo-3-p-tolyl-2,3-dihydrooxazol-4-yl)benzenesulfonamide, and their pharmaceutically acceptable salts, their solvates, their N-oxides, their stereoisomers or their deuterated derivates thereof.

The invention also provides a combination which comprises (1) a pharmaceutical composition as defined above and (3) a therapeutically effective amount of a proton pump inhibitor (PPI). Proton pump inhibitors are a group of drugs whose main action is a pronounced and long-lasting reduction of gastric acid production and, therefore, reduce the gastrointestinal distress.

Typically, the proton pump inhibitor (PPI) is selected from dexlansoprazole, esomeprazole, ilaprazole, lansoprazole, omeprazole, pantoprazole, rabeprazole or mixtures thereof.

In a preferred embodiment, the proton pump inhibitor (PPI) is selected from lansoprazole, omeprazole, pantoprazole or mixtures thereof.

The active compounds in the combination may be administered together in the same pharmaceutical composition or in different compositions intended for separate, simultaneous, concomitant or sequential administration by the same or a different route.

Also provided is a product comprising (1) a pharmaceutical composition as defined above and (3) a therapeutically effective amount of a proton pump inhibitor, as a combined preparation for simultaneous, separate or sequential use in the treatment of a human or animal patient suffering from or susceptible to a pathological condition or disease as defined above.

Also provided is a kit of parts comprising (1) a pharmaceutical composition as defined above, together with instructions for simultaneous, separate or sequential use in combination with (3) a therapeutically effective amount of a proton pump inhibitor, for the treatment of a human or animal patient suffering from or susceptible to a pathological condition or disease as defined above.

Also provided is a package comprising (1) a pharmaceutical composition as defined above and (3) a therapeutically effective amount of a proton pump inhibitor, for simultaneous, separate or sequential use in the treatment of a pathological condition or disease as defined above.

The invention also provides a triple combination which comprises (1) a pharmaceutical composition as defined above, (2) a therapeutically effective amount of a compound that reduces or eliminates flushing as defined above and (3) a therapeutically effective amount of a proton pump inhibitor (PPI) as defined above.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Example 1 - Bulk composition

A 60 kg bulk composition was prepared by mixing the amounts of the ingredients detailed in Table 1.

**Table 1 - Bulk composition**

| **Ingredient** | **Weight (Kg)** | **%** |
|---|---|---|
| Dimethyl fumarate | 15 | 25 |
| Lactose | 18 | 30 |
| Microcrystalline cellulose | 24 | 40 |
| Croscarmellose sodium | 2.4 | 4 |
| Silica colloidal anhydrous | 0.3 | 0.5 |
| Magnesium Stearate | 0.3 | 0.5 |
| **Total** | 60 | 100 |

Dimethyl fumarate, lactose, microcrystalline cellulose, croscarmellose sodium and silica colloidal anhydrous were mixed together to form a blend. The blend was then passed through a 0.8 mm sieve. The sieved blend was mixed again. The resulting was then again passed through a 0.8 mm sieve and finally mixed again.

Magnesium stearate was passed through a 0.5 mm sieve and added to the previous blend, which was mixed to obtain the final blend.

### Example 2 - Tablets

### 2.1. Tablets of the invention

The final blend of Example 1 was divided in two homothetic parts to obtain tablets with different strengths (30 and 120 mg of dimethyl fumarate). 12 Kg of bulk composition were tableted by direct compression with a rotary tableting machine to obtain 100,000 tablet cores with a final weight of 120 mg (30 mg of dimethyl fumarate) and 6.5 mm of diameter.

48 Kg of bulk composition were tableted by direct compression with a rotary tableting machine to obtain 100,000 tablet cores with a final weight of 480 mg (120 mg of dimethyl fumarate) and 11 mm of diameter.

The tablet cores were then coated with an aqueous film coating suspension comprising triethyl citrate, simethicone, talc and titanium dioxide suspended in water and methacrylic acid - ethyl acrylate copolymer (1:1) 30% aqueous dispersion (see amounts in Table 2). In the case of the 120 mg of dimethyl fumarate tablet cores, the coating suspension also contained indigo carmine lake and sodium hydroxide.

**Table 2 - Tablets with lactose**

| | **Ingredient** | **30 mg tablet** | **120 mg tablet** |
|---|---|---|---|
| | | **weight (mg)** | **weight (mg)** |
| Core | Dimethyl fumarate | 30 | 120 |
| | Lactose | 36 | 144 |
| | Microcrystalline cellulose | 48 | 192 |
| | Croscarmellose sodium | 4.8 | 19.2 |
| | Silica colloidal anhydrous | 0.6 | 2.4 |
| | Magnesium Stearate | 0.6 | 2.4 |
| Coating | Methacrylic acid - ethyl acrylate copolymer (1:1) 30% aqueous dispersion | 11.56 | 45.98 |
| | Talc | 5.78 | 22.94 |
| | Triethyl citrate | 1.16 | 4.61 |
| | Simethicone | 0.06 | 0.24 |
| | Titanium dioxide | 0.29 | 4.61 |
| | Indigo carmine Lake | | 1.77 |
| | 1 N Sodium hydroxide solution | | 5.29 |

The enteric-coated tablets described above can be distinguished not only by the colour but also by the size (lower dose - smaller diameter; higher dose - higher diameter), which is beneficial for visually impaired people.

In addition, stability studies were conducted with the enteric-coated tablets described above following stability guidelines of the International Conference on Harmonisation of Technical Requirements for Registration of Pharmaceuticals for Human Use (ICH). Tablets were stored in a climatic chamber at 40°C / 75 % Relative Humidity for 6 months, 30°C / 65 % Relative Humidity for 12 months and at 25°C / 60 % Relative Humidity for 24 months. The appearance, water content, hardness, dissolution and related substances was tested at regular intervals. The results concluded that the tablets were stable. Therefore, no limitation for the storage conditions is needed.

### 2.2. Additional tablets of the invention

In a same manner as described above, 30 and 120 mg dimethyl fumarate enteric-coated tablets were prepared but replacing lactose by fructose, mannitol and dibasic calcium phosphate (CaHPO₄ 2 H₂O) as indicated in Tables 3, 4 and 5. Stability studies were conducted with this tablets at 40°C / 75 % Relative Humidity for 6 months in a climatic chamber. The results concluded that the tablets were stable.

**Table 3 - Tablets with fructose**

| | **Ingredient** | **30 mg tablet** | **120 mg tablet** |
|---|---|---|---|
| | | **weight (mg)** | **weight (mg)** |
| Core | Dimethyl fumarate | 30 | 120 |
| | Fructose | 36 | 144 |
| | Microcrystalline cellulose | 48 | 192 |
| | Croscarmellose sodium | 4.8 | 19.2 |
| | Silica colloidal anhydrous | 0.6 | 2.4 |
| | Magnesium Stearate | 0.6 | 2.4 |
| Coating | Methacrylic acid - ethyl acrylate copolymer (1:1) 30% aqueous dispersion | 11.56 | 45.98 |
| | Talc | 5.78 | 22.94 |
| | Triethyl citrate | 1.16 | 4.61 |
| | Simethicone | 0.06 | 0.24 |
| | Titanium dioxide | 0.29 | 4.61 |
| | Indigo carmine Lake | | 1.77 |
| | 1 N Sodium hydroxide solution (4.54% aqueous dispersion) | | 5.29 |

**Table 4 - Tablets with mannitol**

| | **Ingredient** | **30 mg tablet** | **120 mg tablet** |
|---|---|---|---|
| | | **weight (mg)** | **weight (mg)** |
| Core | Dimethyl fumarate | 30 | 120 |
| | Mannitol | 36 | 144 |
| | Microcrystalline cellulose | 48 | 192 |
| | Croscarmellose sodium | 4.8 | 19.2 |
| | Silica colloidal anhydrous | 0.6 | 2.4 |
| | Magnesium Stearate | 0.6 | 2.4 |
| Coating | Methacrylic acid - ethyl acrylate copolymer (1:1) 30% aqueous dispersion | 11.56 | 45.98 |
| | Talc | 5.78 | 22.94 |
| | Triethyl citrate | 1.16 | 4.61 |
| | Simethicone | 0.06 | 0.24 |
| | Titanium dioxide | 0.29 | 4.61 |
| | Indigo carmine Lake | | 1.77 |
| | 1 N Sodium hydroxide solution | | 5.29 |

**Table 5 - Tablets with dibasic calcium phosphate**

| | **Ingredient** | **30 mg tablet** | **120 mg tablet** |
|---|---|---|---|
| | | **weight (mg)** | **weight (mg)** |
| Core | Dimethyl fumarate | 30 | 120 |
| | Dibasic calcium phosphate | 36 | 144 |
| | Microcrystalline cellulose | 48 | 192 |
| | Croscarmellose sodium | 4.8 | 19.2 |
| | Silica colloidal anhydrous | 0.6 | 2.4 |
| | Magnesium Stearate | 0.6 | 2.4 |
| Coating | Methacrylic acid - ethyl acrylate copolymer (1:1) 30% aqueous dispersion | 11.56 | 45.98 |
| | Talc | 5.78 | 22.94 |
| | Triethyl citrate | 1.16 | 4.61 |
| | Simethicone | 0.06 | 0.24 |
| | Titanium dioxide | 0.29 | 4.61 |
| | Indigo carmine Lake | | 1.77 |
| | 1 N Sodium hydroxide solution | | 5.29 |

### 2.3. Comparative Experiments

In a same manner as described above, 120 mg dimethyl fumarate enteric-coated tablets were prepared but replacing lactose by microcrystalline cellulose. Stability studies were conducted with this tablets at 40°C / 75 % Relative Humidity for 6 months in a climatic chamber. The results concluded that the tablets were not stable.

### Example 3 - Dissolution test

The dissolution rate of dimethyl fumarate in the enteric-coated tablets of the invention described above was determined following a standard dissolution test for solid dosage forms. These dissolution tests are described in the European Pharmacopoeia 6.0, Chapter 2.9.3 and in the US Pharmacopoiea USP36-NF31, Chapter 711.

The dissolution testing was carried out as follows: A USP apparatus II (paddles) with 1 L vessels was used. Bath temperature was set to 37°C±0.5°C and paddle speed to 75 rpm. One tablet is placed in one vessel containing 750 mL 0.1N HCl (pH 1.2) over 2 h. After that the pH is changed to 6.2 by adding 220 mL 0.2 M sodium phosphate buffer (0.05 M phosphate buffer). The tablet is maintained under the buffered pH over 2 h. After that, samples are taken at each sampling time point (every 10 or 20 min). Dimethyl fumarate is detected by UV; cell volume 0.1 cm³, detector wavelength 220 nm, reference wavelength 400-500 nm.

The pharmaceutical compositions of the invention present a dissolution profile similar to Fumaderm ®, despite having reduced the number of active ingredients and having different excipients. Additionally, no relevant differences were observed among the different dissolution profiles of the pharmaceutical compositions of the invention obtained using lactose, fructose, mannitol or dibasic calcium phosphate in the tablet core.

Modifications, which do not affect, alter, change or modify the essential aspects of the pharmaceutical compositions described, are included within the scope of the present invention.

### Further aspects of the invention

1. A pharmaceutical composition comprising (a) dimethyl fumarate, (b) a diluent selected from monosaccharides, disaccharides, starch and starch derivatives, calcium and magnesium inorganic salts, sugar alcohols, and mixtures thereof, (c) microcrystalline cellulose and (d) croscarmellose sodium, wherein the dimethyl fumarate is not covered with a gastro-resistant coating.
2. A pharmaceutical composition according to aspect 1, wherein the diluent (b) is selected from lactose, D-glucose (dextrose), sucrose, fructose, galactose, starch, calcium carbonate, dibasic calcium phosphate, calcium sulphate, magnesium carbonate, isomalt, mannitol, maltitol, sorbitol, xylitol, and mixtures thereof.
3. A pharmaceutical composition according to aspect 1 or aspect 2, wherein the diluent (b) is selected from fructose, lactose, dibasic calcium phosphate, mannitol, and mixtures thereof.
4. A pharmaceutical composition according to any one of aspects 1 to 3, comprising (a) dimethyl fumarate, (b) lactose, (c) microcrystalline cellulose and (d) croscarmellose sodium.
5. A pharmaceutical composition according to any one of aspects 1 to 4, further comprising (e) at least one glidant.
6. A pharmaceutical composition according to aspect 5, wherein the glidant (e) is selected from calcium phosphate, calcium silicate, powdered cellulose, magnesium silicate, magnesium trisilicate, silicon dioxide, talc, colloidal silica, colloidal anhydrous silica and mixtures thereof.
7. A pharmaceutical composition according to any one of aspects 1 to 6, further comprising (f) at least one lubricant.
8. A pharmaceutical composition according to aspect 7, wherein the lubricant (f) is selected from magnesium stearate, calcium stearate, sodium stearyl fumarate, polyethylene glycol, sodium lauryl sulfate, magnesium lauryl sulfate, sodium benzoate, potassium benzoate, light mineral oil, hydrogenated vegetable oils, glycerin monostearate, glyceryl behenate, glyceryl palmitostearate, stearic acid, zinc stearate, and mixtures thereof.
9. A pharmaceutical composition according to any one of aspects 1 to 8, wherein the weight ratio (c) microcrystalline cellulose to (b) diluent is in the range from 2:5 to 5:2.
10. A pharmaceutical composition according to any one of aspects 1 to 9, wherein the composition is administered orally.
11. A pharmaceutical composition according to any one of aspects 1 to 10 in the form of a gastro-resistant (enteric coated) tablet.
12. A pharmaceutical composition according to aspect 11 comprising, based on the total weight of the tablet previous to coating:
   (a) 20-30 wt.% of dimethyl fumarate;
   (b) 25-35 wt.% of a diluent selected from lactose, D-glucose (dextrose), sucrose, fructose, galactose, starch, calcium carbonate, dibasic calcium phosphate, calcium sulphate, magnesium carbonate, isomalt, mannitol, maltitol, sorbitol, xylitol, and mixtures thereof;
   (c) 35-45 wt.% of microcrystalline cellulose;
   (d) 1-10 wt.% of croscarmellose sodium.
13. A pharmaceutical composition according to aspect 12 further comprising (e) at least one glidant.
14. A pharmaceutical composition according to aspect 12 or aspect 13 further comprising (f) at least one lubricant.
15. A pharmaceutical composition according to any one of aspects 12 to 14 comprising, based on the total weight the tablet previous to coating:
   (a) 25 wt.% of dimethyl fumarate;
   (b) 30 wt.% of lactose;
   (c) 40 wt.% of microcrystalline cellulose;
   (d) 4 wt.% of croscarmellose sodium;
   (e) 0.5 wt.% of at least one glidant;
   (f) 0.5 wt.% at least one lubricant.
16. A pharmaceutical composition as defined in any one of aspects 1 to 15 for use in the treatment or prevention of inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis.
17. Use of a pharmaceutical composition as defined in any one of aspects 1 to 15 for the manufacture of a medicament for the treatment or prevention of inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis.
18. A method for treating a subject afflicted with inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis; which comprises administering to said subject a therapeutically effective amount of a pharmaceutical composition as defined in any one of aspects 1 to 15.

## Claims

**1.** A gastro-resistant pharmaceutical composition comprising (a) dimethyl fumarate, (b) a diluent selected from fructose, lactose, dibasic calcium phosphate, mannitol, and mixtures thereof, (c) microcrystalline cellulose and (d) croscarmellose sodium, wherein the composition is coated with a coating comprising triethyl citrate.

**2.** A gastro-resistant pharmaceutical composition according to claim 1, wherein the dimethyl fumarate is not covered with a gastro-resistant coating.

**3.** A gastro-resistant pharmaceutical composition according to claim 1 or claim 2, comprising (a) dimethyl fumarate, (b) lactose, (c) microcrystalline cellulose and (d) croscarmellose sodium.

**5.** A gastro-resistant pharmaceutical composition according to any one of claims 1 to 4, further comprising (e) at least one glidant.

**5.** A gastro-resistant pharmaceutical composition according to claim 4, wherein the glidant (e) is selected from calcium phosphate, calcium silicate, powdered cellulose, magnesium silicate, magnesium trisilicate, silicon dioxide, talc, colloidal silica, colloidal anhydrous silica and mixtures thereof.

**6.** A gastro-resistant pharmaceutical composition according to any one of claims 1 to 5, further comprising (f) at least one lubricant.

**7.** A gastro-resistant pharmaceutical composition according to claim 6, wherein the lubricant (f) is selected from magnesium stearate, calcium stearate, sodium stearyl fumarate, polyethylene glycol, sodium lauryl sulfate, magnesium lauryl sulfate, sodium benzoate, potassium benzoate, light mineral oil, hydrogenated vegetable oils, glycerin monostearate, glyceryl behenate, glyceryl palmitostearate, stearic acid, zinc stearate, and mixtures thereof.

**8.** A gastro-resistant pharmaceutical composition according to any one of claims 1 to 7, wherein the weight ratio (c) microcrystalline cellulose to (b) diluent is in the range from 2:5 to 5:2.

**9.** A gastro-resistant pharmaceutical composition according to any one of claims 1 to 8, wherein the composition is administered orally.

**10.** A gastro-resistant pharmaceutical composition according to any one of claims 1 to 9 in the form of a tablet.

**11.** A gastro-resistant pharmaceutical composition according to claim 10 comprising, based on the total weight of the tablet previous to coating:
(a) 20-30 wt.% of dimethyl fumarate;
(b) 25-35 wt.% of a diluent selected from lactose, fructose, dibasic calcium phosphate, mannitol, and mixtures thereof;
(c) 35-45 wt.% of microcrystalline cellulose;
(d) 1-10 wt.% of croscarmellose sodium.

**12.** A gastro-resistant pharmaceutical composition according to claim 11 further comprising (e) at least one glidant.

**13.** A gastro-resistant pharmaceutical composition according to claim 11 or claim 12 further comprising (f) at least one lubricant.

**14.** A gastro-resistant pharmaceutical composition according to any one of claims 12 to 14 comprising, based on the total weight the tablet previous to coating:
(a) 25 wt.% of dimethyl fumarate;
(b) 30 wt.% of lactose;
(c) 40 wt.% of microcrystalline cellulose;
(d) 4 wt.% of croscarmellose sodium;
(e) 0.5 wt.% of at least one glidant;
(f) 0.5 wt.% at least one lubricant.

**15.** A gastro-resistant pharmaceutical composition as defined in any one of claims 1 to 15 for use in the treatment or prevention of inflammatory or autoimmune diseases or disorders, in particular inflammatory or autoimmune diseases or disorders selected from rheumatoid arthritis, multiple sclerosis (MS), amyotrophic lateral sclerosis, Crohn's disease, ulcerative colitis, systemic lupus erythematosus (SLE), myastenia gravis, acute disseminated encephalomyelitis, idiopathic thrombocytopenic purpura, Sjoegren's syndrome, autoimmune hemolytic anemia (AIHA), type I diabetes or psoriasis.
